# FASCICULE DE BREVET EUROPEEN

(11) **EP 4 423 053 B1**
(45) Date de publication et mention de la délivrance du brevet: **05.08.2026**
(21) Numéro de dépôt: 22801861.0
(22) Date de dépôt: 18.10.2022
(51) Int. Cl.: C07C 67/08, C07C 67/54, C07C 67/58, C07C 69/54

(54) **PROCÉDÉ PERFECTIONNÉ DE FABRICATION D'ACRYLATE DE BUTYLE DE PURETÉ ELEVÉE**
VERBESSERTES VERFAHREN ZUR HERSTELLUNG VON HOCHREINEM BUTYLACRYLAT
IMPROVED METHOD FOR PRODUCING HIGH-PURITY BUTYL ACRYLATE

(30) Priorité: 25.10.2021 FR 2111319
(43) Date de publication de la demande: 04.09.2024
(73) Titulaire: ARKEMA FRANCE, 92800 Puteaux (FR)
(72) Inventeur: TRETJAK, Serge, 57501 Saint Avold Cedex (FR); HILPERT, Camille, 57501 Saint Avold Cedex (FR)
(74) Mandataire: Arkema Patent
(86) Numéro de dépôt international: PCT/FR2022/051965
(87) Numéro de publication internationale: WO 2023/073303

(56) Documents cités:
- EP-A1- 0 609 127
- US-A- 5 767 306

## Description

### DOMAINE TECHNIQUE

La présente invention concerne la fabrication d'acrylate de butyle par estérification directe de l'acide acrylique par du butanol, cette réaction étant catalysée par de l'acide sulfurique. Plus spécifiquement, elle a pour objet un procédé perfectionné de fabrication d'acrylate de butyle, comprenant une étape de valorisation des sous-produits lourds générés au cours de cette fabrication, conduisant à une productivité élevée d'un produit répondant aux normes en matière de pureté et d'acidité, dans des conditions énergétiques optimisées.

### ARRIERE-PLAN TECHNIQUE ET PROBLEME TECHNIQUE

L'estérification de l'acide acrylique est une réaction équilibrée avec génération d'eau qu'il est nécessaire d'éliminer au cours de la réaction pour déplacer l'équilibre dans le sens de la production de l'ester acrylique.

Les problèmes qui se posent lors de la fabrication de l'acrylate de butyle par estérification directe de l'acide acrylique, généralement en présence d'acide sulfurique comme catalyseur, sont le plus souvent liés à la complexité des étapes de purification nécessaires après l'étape réactionnelle pour obtenir un produit de haute pureté, au détriment de la productivité du procédé.

Le procédé industriel, tel que décrit dans le brevet EP 0609127 de la demanderesse, consiste à estérifier l'acide acrylique (AA) par du butanol en excès, en présence d'acide sulfurique. Le mélange réactionnel en fin de réaction comprend de l'acrylate de butyle, de l'acide acrylique résiduel, du sulfate acide de butyle, des traces d'acide sulfurique et différentes impuretés résultant de réactions secondaires. Ce mélange réactionnel est ensuite soumis à une étape de neutralisation et de lavage à l'eau qui a pour objet d'éliminer les impuretés dites acides : acide sulfurique résiduel, sulfate acide de butanol et acide acrylique. Ce mélange exempt d'impuretés acides est soumis à différentes étapes de purification, qui conduisent à la récupération de l'acrylate de butyle purifié. Une des étapes dite d'étêtage consiste notamment à distiller le butanol et les sous- produits légers. Le butanol peut être ainsi recyclé à la réaction d'estérification.

L'étape finale de purification de l'acrylate de butyle consiste à envoyer le mélange contenant de l'ester débarrassé des produits légers dans une dernière colonne de distillation d'où il sort en tête, purifié des sous-produits lourds qui eux se retrouvent en pied de la colonne de distillation et sont ensuite concentrés dans un évaporateur. Le produit de tête de cet évaporateur, à savoir l'acrylate de butyle (ABU), est renvoyé en pied de la colonne de rectification ce qui permet d'une part de le récupérer comme produit fini mais également de maintenir la température du fond de colonne assez basse pour éviter des problèmes d'encrassement liés au caractère thermo sensible de ce monomère.

Le résidu de l'évaporateur, outre les dérivés de Michael et quelques pourcents de monomères libres, et également quelques pourcents d'inhibiteurs de polymérisation, accumulés au fur et à mesure de l'ensemble des étapes de purification, comme majoritairement la phénothiazine sous sa forme libre ou d'adduit de l'AA ou de l'ABU, ainsi que des composés lourds de nature polymérique plus ou moins solubles dans le milieu. En général ce résidu est éliminé par incinération, ce qui entraîne une perte de rendement importante.

Parmi les sous-produits générés selon les réactions secondaires, on peut citer les produits légers tels que l'acétate de butyle, le propionate de butyle, l'éther dibutylique, l'acrylate d'isobutyle ou les produits lourds comme le maléate de dibutyle.

Les composés « lourds » issus de réactions d'addition de Michael se forment spontanément dans les unités de production d'acrylate de butyle. Ces réactions parasites sont favorisées par des températures élevées rencontrées en particulier dans les pieds de colonnes à distiller de ces unités. Ainsi l'acide acrylique, le butanol n'ayant pas encore réagi ou l'eau de réaction s'ajoutent à la double liaison de l'acrylate de butyle pour former principalement :
- l'acryloxypropionate de butyle (AA/ABU) par addition de l'acide acrylique (AA) sur l'acrylate de butyle (ABU) ;
- l'hydroxypropionate de butyle (HPB) par addition d'eau sur l'acrylate de butyle ;
- le butoxypropionate de butyle (BPB) par addition de butanol sur l'acrylate de butyle.

Une polyaddition ou la formation de composés mixtes est également possible.

Une des caractéristiques des sous-produits lourds est que leur point d'ébullition se trouve au-dessus des points d'ébullition de l'acide acrylique, du butanol et de l'acrylate de butyle.

Comme leur volatilité est faible, ils s'accumulent en fond de la dernière colonne de distillation, en pied de l'évaporateur servant à concentrer ce résidu.

Différentes solutions ont été proposées pour la valorisation de ces sous-produits lourds contenant encore les inhibiteurs de polymérisation.

Le document CN1063678 propose des méthodes de traitement des oxy-esters formés lors de la synthèse d'acrylate de butyle en utilisant des catalyseurs acides protiques comme l'acide sulfurique ou l'acide para toluène sulfonique. Des composés tels des phtalates peuvent également être ajoutés, comme décrit dans le document US 4293347.

Le désavantage de ces méthodes de craquage est que le produit résiduel est visqueux et contient des solides. US 6617470 propose d'utiliser comme catalyseurs, des acides arylsulfoniques comme l'acide dodécylsulfonique, ce qui supprime la formation de solides dans le résidu de pied.

Le document US 2011/0230675 propose d'ajouter de l'eau en continu lorsque le craquage est effectué par catalyse acide, afin d'éviter la formation de dépôt solide.

Enfin le document CN102173990 propose d'ajouter dans l'alimentation du craqueur des sels de cuivre afin de faciliter le traitement ultérieur du résidu ultime de craquage.

Le document US 5767306 décrit un procédé de fabrication d'acrylate de butyle par estérification directe de l'AA par du butanol en présence d'acide sulfurique comme catalyseur, et d'au moins un inhibiteur de polymérisation, comprenant des étapes de distillation permettant de séparer un flux contenant des sous-produits lourds qui est concentré sur un évaporateur à flux tombant générant deux flux : un flux pour alimenter un craqueur et un flux contenant un résidu avec les inhibiteurs de polymérisation.

La société demanderesse a décrit dans sa demande FR2101402 un schéma de procédé combinant une colonne à soutirage latéral, un craqueur thermique ou thermique et catalytique et un système de décanteur et de lavage pour traiter les produits issus du craqueur pour la synthèse d'ester acrylique tel l'acrylate de 2-éthylhexyle. Cette combinaison est rendue nécessaire car, dans ces procédés catalysés par des résines acides, les impuretés dites acides ne sont, d'une part, pas éliminées lors d'une opération de neutralisation préalablement à la section de purification et, d'autre part, sont également générées lors du craquage. Sans la combinaison : colonne à soutirage latéral - décanteur (voir exemple 2), il n'est pas possible d'obtenir un acrylate de 2-éthylhexyle purifié conforme aux spécifications.

Sans que la demanderesse soit tenue à une quelconque explication, elle pense que c'est l'interaction entre la phénothiazine et le catalyseur acide lors d'un craquage catalytique et thermique qui est en grande partie responsable de la formation de solide dans le résidu ultime du craqueur.

La demande FR2108885 met en œuvre un craqueur thermique sans catalyseur, ce qui permet de supprimer cette interaction avec succès. Le rendement de craquage reste cependant plus faible que celui obtenu lors d'un craquage thermique et catalytique.

Pour résoudre le problème de l'encrassement des installations, le brevet FR 2901272 met en œuvre une opération de distillation sous vide à une température relativement basse, sous atmosphère inerte, éventuellement en présence d'un dispersant, afin de limiter dans le produit de distillation la teneur en phénothiazine à une valeur inférieure à 100 ppm, avant de réaliser un craquage thermique et catalytique à pression atmosphérique sous atmosphère inerte.

L'opération d'élimination de la phénothiazine nécessite dans ce procédé un bouilleur, une colonne à distiller opérée à pression réduite, un condenseur, un équipement de reflux et un dévésiculeur et une injection d'un gaz inerte. La conjonction de cette distillation pour éliminer la phénothiazine et d'un craquage catalytique à pression atmosphérique, en présence d'un inerte permet d'éviter la formation de solides dans le résidu avec un taux de craquage très satisfaisant. Ainsi le procédé tel qu'envisagé comporte une colonne de rectification, un évaporateur en pied de cette colonne, la distillation sous pression réduite sous gaz inerte et enfin le craquage thermique et catalytique sous gaz inerte également.

Il subsiste un besoin de simplifier ce schéma de procédé, qui reste laborieux à mettre en œuvre.

Il a maintenant été trouvé qu'en remplaçant l'évaporateur en pied de colonne de rectification et la distillation sous pression réduite et sous atmosphère inerte, par un seul évaporateur et son système de condensation étagé, on diminue significativement la formation de solide dans le résidu du craqueur, tout en simplifiant les équipements mis en œuvre, dans un procédé permettant l'obtention d'acrylate de butyle de haute pureté.

### RESUME DE L'INVENTION

La présente invention décrit un système d'évaporation permettant, par le recyclage de l'acrylate de butyle, de garder une température de fond de colonne de rectification compatible avec le caractère thermosensible de l'acrylate de butyle et d'envoyer les adduits de Michael au craqueur thermique et catalytique, en purgeant en pied de cet évaporateur les composés très lourds et les inhibiteurs de polymérisation, pour un procédé permettant l'obtention d'acrylate de butyle de haute pureté, comme décrit dans le brevet EP 0609127 pour la partie réactionnelle. Elle complète également le schéma de purification décrit dans ce brevet en associant un évaporateur placé au bas de la colonne de purification de l'acrylate de butyle avec son système de condensation étagé et décrit le recyclage des produits de tête issus du craquage dans le procédé.

L'invention a pour objet un procédé de fabrication d'acrylate de butyle par estérification directe de l'acide acrylique par du butanol en excès en présence d'acide sulfurique comme catalyseur et au moins un inhibiteur de polymérisation, conduisant à l'obtention d'un mélange réactionnel brut contenant de l'acrylate de butyle, acide acrylique et butanol résiduels, sulfate acide de butyle, des traces d'acide sulfurique et des impuretés résultant des réactions secondaires, ledit procédé comprenant des étapes de neutralisation et de lavage à l'eau conduisant à l'obtention d'un mélange réactionnel exempt d'impuretés dites acides, caractérisé en ce que ledit mélange réactionnel lavé d'impuretés acides est soumis au moins aux étapes i), ii) et iii) suivantes:
i) étêtage dans une colonne de distillation permettant d'obtenir:
   - en tête un flux composé essentiellement des réactifs non réagis ;
   - en pied un flux comprenant l'ester recherché et des sous-produits lourds ;
ii) on soumet le flux de pied de la colonne d'étêtage à une colonne de rectification permettant de séparer:
   - en tête l'ester recherché purifié;
   - en pied un flux contenant des sous-produits lourds, qui est concentré sur un évaporateur à couche mince avec un flux de tête refroidi en deux étapes successives de condensation afin de recycler à la colonne de rectification les composés légers présents, d'éliminer un résidu final comportant le ou les inhibiteurs de polymérisation et d'envoyer au craqueur les adduits de Michael avec une teneur réduite en inhibiteur(s), en particulier phénothiazine, à moins de 1000 ppm, de préférence à moins de 300 ppm;
iii) le flux contenant les adduits de Michael qui sont envoyés vers un craqueur thermique et catalytique permettant d'obtenir:
   - en tête les produits nobles issus du craquage qui sont recyclés à la réaction ;
   - en pied le résidu ultime qui est envoyé à une station de traitement des déchets.

La présente invention permet de surmonter les inconvénients de l'état de l'art. Elle fournit plus particulièrement un procédé permettant d'obtenir un acrylate de butyle de haute pureté ayant comme spécifications une pureté en ester supérieure à 99,5%, intégrant un procédé d'élimination des inhibiteurs de polymérisation optimisé, permettant d'effectuer un craquage des adduits de Michael en réactifs (acide acrylique et alcool) et en produit fini, augmentant ainsi la productivité du procédé en limitant la quantité de résidu à éliminer.

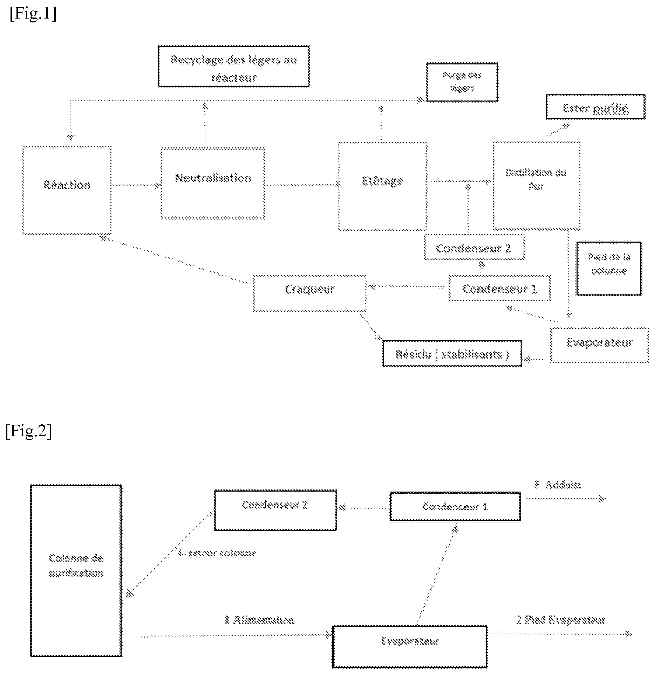

D'autres caractéristiques et avantages de l'invention ressortiront mieux à la lecture de la description détaillée qui suit, en référence aux Figure 1 et 2 annexées.
[Fig. 1] : schéma global du procédé de synthèse de l'acrylate de butyle selon l'invention, avec un échangeur en pied de la colonne de purification de l'acrylate de butyle.
[Fig. 2] : procédé selon l'invention d'évaporation avec son système de condensation étagé.

### DESCRIPTION DETAILLEE DE L'INVENTION

L'invention a pour objet un procédé de fabrication d'acrylate de butyle par estérification directe de l'acide acrylique par du butanol en excès, en présence d'acide sulfurique comme catalyseur, et au moins un inhibiteur de polymérisation, conduisant à l'obtention d'un mélange réactionnel brut contenant de l'acrylate de butyle, acide acrylique et butanol résiduels, sulfate acide de butyle, des traces d'acide sulfurique et des impuretés résultant des réactions secondaires. Selon diverses réalisations, ledit procédé comprend les caractères suivants, le cas échéant combinés.

Après l'étape d'estérification, le procédé selon l'invention comprend des étapes de neutralisation et de lavage à l'eau conduisant à l'obtention d'un mélange réactionnel débarrassé des impuretés dites acides : acide sulfurique, sulfate acide de butyle, dimère d'acide acrylique et acide acrylique résiduel.

De manière caractéristique, le mélange réactionnel lavé d'impuretés acides comme décrit cidessus est soumis au moins aux étapes i), ii) et iii) suivantes :
i) étêtage dans une colonne de distillation permettant d'obtenir :
   - en tête un flux composé essentiellement des réactifs non réagis ;
   - en pied un flux comprenant l'ester recherché et des sous-produits lourds ;
ii) on soumet le flux de pied de la colonne d'étêtage à une colonne de rectification permettant de séparer :
   - en tête l'ester recherché purifié ;
   - en pied un flux contenant des sous-produits lourds et les inhibiteurs présents à une teneur de l'ordre de 1 à 3% dans les étapes de purification précédentes, qui est concentré sur un évaporateur à couche mince générant trois flux :
   - un flux de tête d'acrylate de butyle, refroidi en deux étapes successives de condensation, à recycler à la colonne de rectification ;
   - un flux d'adduits de Michael pour alimenter le craqueur ;
   - un flux contenant de résidu contenant les inhibiteurs de polymérisation.
iii) on soumet le flux d'adduits de Michael à un traitement thermique et catalytique effectué dans un craqueur permettant de séparer :
   - en tête, un flux de produits valorisables recyclé à la réaction ;
   - en pied, un résidu envoyé vers une station de traitement des déchets.

Température de réaction et pression de l'évaporateur sont reliées de façon à ce que l'on élimine par évaporation l'acrylate de butyle et les adduits de Michael.

Selon un mode de réalisation, l'évaporation est effectuée dans une gamme de températures de 80°C à 100°C et plus spécialement entre 90°C et 100°C.

Selon un mode de réalisation, la pression maintenue de l'évaporateur est comprise entre 800 Pa à 2000 Pa.

Selon un mode de réalisation, le flux de tête de cet évaporateur est refroidi en deux étapes successives :
-- une condensation partielle dans une gamme de température de 50 à 80°C et plus spécialement 60°C-70°C à la même pression que celle de l'évaporateur pour obtenir d'une part, un flux liquide d'adduits de Michael, ayant une teneur en inhibiteurs réduite inférieure à1000ppm allant alimenter le craqueur, et d'autre part, un flux de vapeur, et

- une condensation de ce flux de vapeur dans une gamme de température de 20°C à 40°C et plus spécialement 20°C-30°C avant reprise de ce flux liquide par une pompe et alimentation de la colonne de rectification.

La composition massique du produit d'alimentation de l'évaporateur en pied de la colonne de rectification dans le cas de la fabrication de l'acrylate de butyle en présence de phénothiazine, comme inhibiteur majoritairement mis en œuvre, est la suivante :
- Acrylate de butyle (40-50%)
- Hydroxypropionate de butyle (HPB) : 0,5-1,5%
- Butoxypropionate de butyle (BPB) : 50-70%
- Acryloxypropionate de butyle (AA/ABU) : 2-3%
- Dibutylmaléate : 1-3%
- Phénothiazine : 1-3%.

Le temps de séjour dans l'évaporateur est de l'ordre de la minute.

En référence à la Figure 1, qui représente le mode d'invention préféré, la section d'étêtage comporte une colonne à distiller possédant un équivalent de 10 et 30 plateaux théoriques de préférence 10 à 15 étages théoriques. Les internes utilisés pour la colonne peuvent être des plateaux à clapets ou des plateaux perforés à déversoir, des plateaux à courants croisés comme les Dual Flow les Ripple Trays, les Turbo Grid Shell, ou du garnissage ordonné comme du garnissage structuré tel le Mellapack 250X de Sulzer.

L'alimentation de la colonne d'étêtage est réalisée au tiers supérieur de cette colonne, de préférence entre les plateaux théoriques 3 à 10 comptées à partir de la tête de la colonne. Le flux de tête de la colonne comprend essentiellement les réactifs non réagis. Ce flux valorisable est recyclé à la réaction.

La colonne fonctionne avec un taux de reflux (débit de liquide condensé retourné à la colonne/ débit recyclé à la réaction) compris entre 4/1 à 1/1, de préférence 3/1. Avantageusement, on introduit de 50 à 5000 ppm d'inhibiteur de polymérisation dans le système de purification selon le procédé de l'invention.

Comme inhibiteurs de polymérisation utilisables, on peut citer par exemple la phénothiazine (PTZ), l'hydroquinone (HQ), l'éther mono méthylique d'hydroquinone (EMHQ), le di-tert-butyl para-crésol (BHT), la paraphénylène diamine, le TEMPO (2,2,6,6-tétraméthyl-1-piperidinyloxy), le di-tert-butylcatéchol, ou les dérivés du TEMPO, tel que le OH-TEMPO, seuls ou leurs mélanges en toutes proportions, à des teneurs dans le milieu de réaction pouvant être comprises entre 50 ppm et 5000 ppm, éventuellement en présence d'air appauvri, mais généralement à des teneurs comprises entre 150 ppm et 1000 ppm. L'ajout des inhibiteurs de polymérisation peut se faire à différents endroits, avec l'introduction des réactifs ou en tête de colonne de distillation.

Pour rendre les inhibiteurs plus efficaces il convient d'injecter en pied de colonne de l'oxygène, de l'air ou de l'air dit appauvri à 7% O2. De façon préférée la quantité d'oxygène injectée correspond à une teneur de 0,2% à 0,5% rapportée à la quantité de vapeur organique dans la colonne.

La colonne peut opérer sous vide, afin de minimiser l'exposition thermique des composés thermosensibles au sein de la colonne. Avantageusement, la colonne d'étêtage fonctionne sous un vide allant de 1000 Pa à 30000 Pa.

Le flux de pied alimente de préférence la colonne permettant d'obtenir l'ester purifié en pied de colonne entre le plateau théorique 6 à 9.

La colonne de distillation du pur comporte un équivalent de 2 et 15 plateaux théoriques, de préférence 6 à 12 étages théoriques. Les internes utilisés pour la colonne peuvent être des plateaux à clapets ou des plateaux perforés à déversoir, des plateaux à courants croisés comme les Dual Flow les Ripple Trays, les Turbo Grid Shell, ou du garnissage ordonné comme du garnissage structuré tel le Mellapack 250X de Sulzer.

Le flux de tête de la colonne est constitué de l'acrylate de butyle de haute pureté ayant comme spécifications une pureté en ester supérieure à 99,5%.

La colonne fonctionne avec un taux de reflux (débit de liquide condensé retourné à la colonne/ débit de pur) compris entre un ratio de 1/8 à 1/1 de préférence 1/4. Comme la colonne d'étêtage, celle-ci est stabilisée et de l'air ou de l'air appauvri (7% O2) est injecté en bas de colonne. La colonne peut opérer sous vide, afin de minimiser l'exposition thermique des composés thermosensibles au sein de la colonne. Avantageusement, la colonne de pur fonctionne sous un vide allant de 1000 Pascal à 20000 Pascal.

Avantageusement la température de fonctionnement est comprise entre 50°C et 160 °C.

Le flux de pied est concentré sur un évaporateur à couche mince, adapté aux produits visqueux, encrassants et aux liquides souillés. D'autres technologies telles les échangeurs plaque-calandre ne répondent pas à ce besoin car le temps de séjour dans ces derniers sont trop longs.

L'évaporation est effectuée dans une gamme de température de 80°C à 100°C et plus spécialement entre 90°C et 100°C, dans une gamme de pression comprise entre 800 Pa à 2000 Pa.

Selon un mode de réalisation, la teneur en inhibiteur de polymérisation dans le flux de pied de la colonne de rectification contenant le flux d'adduits de Michael est inférieure à 3% dans ce flux de pied.

Le flux d'adduit de tête peut alors alimenter un craqueur tel que décrit dans FR 2901272. Ce craqueur est un réacteur à recirculation forcée comprenant un échangeur externe. La température du milieu réactionnelle est comprise entre 160° et 180°C. La pression dans ce réacteur est maintenue à la pression atmosphérique. Le produit de pied constitue le résidu ultime et il est envoyé vers la filière adéquate. Le produit de tête condensé à une température de 20°C à 30°C est envoyé au réacteur. Il n'est pas nécessaire d'injecter dans ce réacteur de l'air ou de l'air appauvri car la teneur résiduelle de stabilisant dans ce flux d'adduit est suffisante pour éviter les problèmes de formation de solides.

Les exemples ci-après illustrent la présente invention sans toutefois en limiter la portée.

### PARTIE EXPERIMENTALE

Dans les exemples, les pourcentages sont indiqués en poids sauf indication contraire et les abréviations suivantes ont été utilisées :
PTZ : Phénothiazine
ABU : acrylate de butyle
BuOH: butanol
BPB : butoxypropionate de butyle
DBE : éther dibutylique
APB : Acryloxypropionate de butyle

### Exemple 1. Essai avec un évaporateur à couche mince

Un évaporateur à couche mince DV210/1m2 commercialisé chauffé par de la vapeur 400 kPa, fonctionnant à une pression de 130 Pa est alimenté à raison de 50kg/h par un mélange comprenant : ABU : 9% ; BuOH: 3,5% ; BPB : 67 % ; APB : 5,7% ; PTZ : 1,8% ; complément à 100% : résidu lourd.

Le produit de tête (40 kg/h) est condensé à 20 °C dans un échangeur tubulaire de 4m2. Il est stabilisé par un ajout d'une solution de 250g /h d'Acrylate de butyle contenant 2% de phénothiazine. Ce produit de tête comprend : 12,5% ABU ; 75 % BPB ; 3% Butanol et 900 ppm de Phénothiazine. Le produit de pied de l'évaporateur contenant la PTZ (6%) ne présente pas de particules. Après une semaine de fonctionnement, l'échangeur et les circuits sont propres.

### Exemple 2. Essai avec un évaporateur plaque calandre

Un évaporateur plaque calandre Vahterus type1 PSHE4/2HA-40/1/1 1,2m2 commercialisé par la société Kapp chauffé par de la vapeur 400 kPa, fonctionnant à une pression de 130 Pa est alimenté à raison de 50kg/h par un mélange comprenant : ABU : 9% ; BuOH : 3,5% ; BPB : 67 % ; APB : 5,7% ; PTZ : 1,8% ; complément à 100% : résidu lourd. Le temps de séjour de la phase liquide dans l'échangeur est de 20 min.

Le produit de pied de l'évaporateur contenant la PTZ (6%) ne présente pas de particules. Cependant après deux jours de fonctionnement, l'échangeur est encrassé.

### Exemple 3. Procédé suivant l'invention

Le procédé d'évaporation simulé sous Aspen V10 est représenté sur la figure 2 annexée. Le tableau 1 ci-dessous indique les conditions de travail utilisées, et les compositions des différentes phases.

Cette simulation montre, d'une part, que l'évaporateur dans ces conditions génère une phase gazeuse dans laquelle l'inhibiteur est absent et, d'autre part, que l'on peut obtenir un flux d'adduits liquide et un flux d'acrylate de butyle qui vont pouvoir être envoyés vers le craqueur et à l'alimentation de la colonne.

**[Tableau 1]**

| | Unités | Alimentation Evaporateur | Phase liquide retour colonne | Adduits | Pied Evaporateur |
|---|---|---|---|---|---|
| Phase | | Liquide | Liquide | Liquide | Liquide |
| Température | °C | 109,6 | 20 | 68,5 | 95 |
| Pression | Pascal | 17330 | 100000 | 1000 | 1000 |
| Débit Massique | Kg/h | 14000 | 5880 | 6529 | 1591 |
| Fraction Massique | | | | | |
| ABU | % | 43 | 89 | 11 | 2 |
| BuOH | % | 0 | 0 | 0 | 0 |
| DBE | ppm | 28 | 61 | 5 | 1 |
| PTZ | ppm | 12000 | 0,3 | 700 | 102300 |
| BPB | % | 56 | 10,9 | 8,9 | 8,7 |

## Revendications

1. Procédé de fabrication d'acrylate de butyle par estérification directe de l'acide acrylique par du butanol en présence d'acide sulfurique comme catalyseur, et d'au moins un inhibiteur de polymérisation, conduisant à l'obtention d'un mélange réactionnel brut contenant de l'acrylate de butyle, acide acrylique et butanol résiduels, sulfate acide de butyle, des traces d'acide sulfurique et des impuretés résultant des réactions secondaires, ledit procédé comprenant des étapes de neutralisation et de lavage à l'eau conduisant à l'obtention d'un mélange réactionnel exempt d'impuretés acides, **caractérisé en ce que** ledit mélange réactionnel lavé d'impuretés acides est soumis au moins aux étapes suivantes :
i) étêtage dans une colonne de distillation permettant d'obtenir :
- en tête un flux composé essentiellement des réactifs non réagis ;
- en pied un flux comprenant l'ester recherché et des sous-produits lourds ;
ii) on soumet le flux de pied de la colonne d'étêtage à une colonne de rectification permettant de séparer :
- en tête l'acrylate de butyle purifié ;
- en pied un flux contenant des sous-produits lourds, qui est concentré sur un évaporateur à couche mince générant trois flux :
∘ un flux de tête d'acrylate de butyle, refroidi en deux étapes successives de condensation, avant recyclage à la colonne de rectification ;
∘ un flux d'adduits de Michael pour alimenter le craqueur ;
∘ un flux contenant de résidu contenant les inhibiteurs de polymérisation.
iii) le flux contenant les adduits de Michael qui sont envoyés vers un craqueur thermique et catalytique permettant d'obtenir :
- en tête les produits nobles issus du craquage qui sont recyclés à la réaction;
- en pied le résidu ultime qui est envoyé à une station de traitement des déchets.

2. Procédé selon la revendication 1, dans lequel lesdites impuretés acides sont l'acide sulfurique, le sulfate acide de butyle, le dimère d'acide acrylique et l'acide acrylique résiduel.

3. Procédé selon l'une quelconque des revendications 1 et 2, dans lequel l'inhibiteur de polymérisation est choisi parmi : la phénothiazine (PTZ), l'hydroquinone (HQ), l'éther mono méthylique d'hydroquinone (EMHQ), le di-tert-butyl para-crésol (BHT), la paraphénylène diamine, le TEMPO (2,2,6,6-tétraméthyl-1-piperidinyloxy), le di-tert-butylcatéchol, le OH-TEMPO, ou leurs mélanges en toutes proportions.

4. Procédé selon l'une quelconque des revendications 1 à 3, dans lequel l'inhibiteur de polymérisation est utilisé dans le milieu de réaction ou dans les étapes de purification à des teneurs comprises entre 50 ppm et 5000 ppm, de préférence comprises entre 150 ppm et 1000 ppm.

5. Procédé selon l'une quelconque des revendications 1 à 4, dans lequel l'inhibiteur de polymérisation est ajouté à différents endroits, avec les réactifs ou en tête de colonne de distillation.

6. Procédé selon l'une quelconque des revendications 1 à 5, dans lequel l'évaporation est effectuée dans une gamme de températures de 80°C à 100°C, de préférence entre 90°C et 100°C, et à une pression comprise entre 800 Pa à 2000 Pa.

7. Procédé selon l'une quelconque des revendications 1 à 6, dans lequel le flux de tête de l'évaporateur est refroidi en deux étapes successives :
a. une condensation partielle dans une gamme de température de 50 à 80°C et plus spécialement 60°C-70°C à la même pression que celle de l'évaporateur pour obtenir d'une part, un flux liquide d'adduits de Michael, ayant une teneur en inhibiteurs inférieure à 1000 ppm, allant alimenter le craqueur, et d'autre part, un flux de vapeur, et
b. une condensation de ce flux de vapeur dans une gamme de température de 20°C à 40°C et plus spécialement 20°C-30°C avant reprise de ce flux liquide par une pompe et alimentation de la colonne de rectification.

8. Procédé selon l'une quelconque des revendications 1 à 6, dans lequel le craquage est effectué à une température de 160°C à 180°C à pression atmosphérique.

9. Procédé selon l'une quelconque des revendications 1 à 8, dans lequel l'acrylate de butyle obtenu a une pureté supérieure à 99,5%.

10. Procédé selon l'une quelconque des revendications 1 à 9, dans lequel la teneur en inhibiteur de polymérisation dans le flux de pied de la colonne de rectification contenant le flux d'adduits de Michael est inférieure à 3%.

## Patentansprüche

1. Verfahren zur Herstellung von Butylacrylat durch direkte Veresterung von Acrylsäure durch Butanol in Gegenwart von Schwefelsäure als Katalysator und mindestens einem Polymerisationsinhibitor, was zum Erhalten eines rohen Reaktionsgemisches führt, das Butylacrylat, restliche Acrylsäure und restliches Butanol, Butylhydrogensulfat, Schwefelsäurespuren und aus Nebenreaktionen resultierende Verunreinigungen enthält, wobei das Verfahren Schritte zum Neutralisieren und Waschen mit Wasser umfasst, die zum Erhalten eines Reaktionsgemisches führen, das frei von sauren Verunreinigungen ist, **dadurch gekennzeichnet, dass** das von sauren Verunreinigungen gewaschene Reaktionsgemisch mindestens den folgenden Schritten unterzogen wird:
i) Abtrennen der Kopffraktion in einer Destillationskolonne, was es ermöglicht, Folgendes zu erhalten:
- am Kopf einen Strom, der im Wesentlichen aus nicht umgesetzten Edukten besteht;
- am Sumpf einen Strom, der den angestrebten Ester und schwerflüchtige Nebenprodukte umfasst;
ii) der Sumpfstrom der Kolonne zum Abtrennen der Kopffraktion wird einer Rektifikationskolonne unterzogen, was es ermöglicht, Folgendes abzutrennen:
- am Kopf das gereinigte Butylacrylat;
- am Sumpf einen schwerflüchtige Nebenprodukte enthaltenden Strom, der in einem Filmverdampfer konzentriert ist, der drei Ströme erzeugt:
∘ einen Kopfstrom von Butylacrylat, der in zwei aufeinanderfolgenden Kondensationsschritten abgekühlt wird, bevor er zur Rektifikationskolonne zurückgeführt wird;
∘ einen Strom von Michael-Addukten zur Speisung des Crackers;
∘ einen Strom, der Rückstand enthält, welcher die Polymerisationshemmer enthält;
iii) der Strom, der die Michael-Addukte enthält, die einem thermischen und katalytischen Cracker zugeführt werden, was es ermöglicht, Folgendes zu erhalten:
- am Kopf einen Strom aus verwertbaren Produkten aus dem Cracken, die zur Reaktion zurückgeführt werden;
- am Sumpf den letztendlichen Rückstand, der einer Abfallbehandlungsstation zugeführt wird.

2. Verfahren nach Anspruch 1, wobei die sauren Verunreinigungen Schwefelsäure, Butylhydrogensulfat, Acrylsäuredimer und restliche Acrylsäure sind.

3. Verfahren nach einem der Ansprüche 1 und 2, wobei der Polymerisationsihibitor aus Phenothiazin (PTZ), Hydrochinon (HQ), Hydrochinonmonomethylether (HOME), Di-tert-butyl-para-kresol (BHT), para-Phenylendiamin, TEMPO (2,2,6,6-Tetramethyl-1-piperidinyloxy), Di-tert-butylcatechol, OH-TEMPO oder Mischungen davon in beliebigem Verhältnis ausgewählt wird.

4. Verfahren nach einem der Ansprüche 1 bis 3, wobei der Polymerisationsinhibitor im Reaktionsmedium oder in den Reinigungsschritten in Gehalten zwischen 50 ppm und 5000 ppm, vorzugsweise zwischen 150 ppm und 1000 ppm, verwendet wird.

5. Verfahren nach einem der Ansprüche 1 bis 4, wobei der Polymerisationsinhibitor an verschiedenen Stellen mit den Reagenzien oder am Kopf der Destillationskolonne zugegeben wird.

6. Verfahren nach einem der Ansprüche 1 bis 5, wobei die Verdampfung in einem Temperaturbereich von 80 °C bis 100 °C, vorzugsweise zwischen 90 °C und 100 °C, und bei einem Druck zwischen 800 Pa und 2000 Pa durchgeführt wird.

7. Verfahren nach einem der Ansprüche 1 bis 6, wobei der Kopfstrom des Verdampfers in zwei aufeinanderfolgenden Schritten abgekühlt wird:
a. teilweise Kondensation in einem Temperaturbereich von 50 bis 80 °C und insbesondere 60 °C bis 70 °C bei dem gleichen Druck wie der Verdampfer, um einerseits einen Flüssigkeitsstrom von Michael-Addukten mit einem Gehalt an Inhibitoren von weniger als 1000 ppm, der dem Cracker zugeführt wird, und andererseits einen Dampfstrom zu erhalten, und
b. Kondensation dieses Dampfstroms in einem Temperaturbereich von 20 °C bis 40 °C und insbesondere 20 °C bis 30 °C, bevor dieser Flüssigkeitsstrom mit einer Pumpe aufgenommen und in die Rektifikationskolonne eingespeist wird.

8. Verfahren nach einem der Ansprüche 1 bis 6, wobei das Cracken bei einer Temperatur von 160 °C bis 180 °C bei Normaldruck durchgeführt wird.

9. Verfahren nach einem der Ansprüche 1 bis 8, wobei das erhaltene Butylacrylat eine Reinheit von mehr als 99,5 % aufweist.

10. Verfahren nach einem der Ansprüche 1 bis 9, wobei der Gehalt an Polymerisationsinhibitor im Sumpfstrom der Rektifikationskolonne, die den Strom von Michael-Addukten enthält, kleiner als 3 % ist.

## Claims

1. Process for producing butyl acrylate by direct esterification of acrylic acid with butanol in the presence of sulfuric acid as catalyst and of at least one polymerization inhibitor, resulting in production of a crude reaction mixture containing butyl acrylate, residual acrylic acid and residual butanol, butyl hydrogen sulfate, traces of sulfuric acid and impurities resulting from side reactions, said process comprising neutralization and washing steps with water leading to the production of a reaction mixture free of acidic impurities, **characterized in that** said reaction mixture washed of acidic impurities is subjected at least to the following steps:
i) topping in a distillation column to obtain:
- at the top, a stream composed essentially of unreacted reactants;
- at the bottom, a stream comprising the desired ester and heavy by-products;
ii) the bottom stream from the topping column is subjected to a rectification column to separate:
- at the top, purified butyl acrylate;
- at the bottom, a stream containing heavy by-products, which is concentrated on a thin-film evaporator generating three streams:
∘ a butyl acrylate top stream, cooled in two successive condensation steps, before recycling to the rectification column;
∘ a Michael adduct stream for feeding the cracker;
∘ a stream containing residue containing polymerization inhibitors;
iii) the stream containing Michael adducts that are sent to a thermal and catalytic cracker to obtain:
- at the top, the noble products resulting from the cracking which are recycled to the reaction;
- at the bottom, the ultimate residue that is sent to a waste treatment plant.

2. Process according to Claim 1, wherein said acidic impurities are sulfuric acid, butyl hydrogen sulfate, acrylic acid dimer and residual acrylic acid.

3. Process according to either one of Claims 1 and 2, wherein the polymerization inhibitor is chosen from: phenothiazine (PTZ), hydroquinone (HQ), hydroquinone monomethyl ether (HQME), di-tert-butyl para-cresol (BHT), para-phenylenediamine, TEMPO (2,2,6,6-tetramethyl-1-piperidinyloxy), di-tert-butylcatechol, OH-TEMPO, or mixtures thereof in all proportions.

4. Process according to any one of Claims 1 to 3, wherein the polymerization inhibitor is used in the reaction medium or in the purification steps at contents between 50 ppm and 5000 ppm, preferably between 150 ppm and 1000 ppm.

5. Process according to any one of Claims 1 to 4, wherein the polymerization inhibitor is added at different locations, with the reactants or at the top of the distillation column.

6. Process according to any one of Claims 1 to 5, wherein the evaporation is carried out in a temperature range of 80°C to 100°C, preferably between 90°C and 100°C, and at a pressure of between 800 Pa and 2000 Pa.

7. Process according to any one of Claims 1 to 6, wherein the top stream from the evaporator is cooled in two successive steps:
a. a partial condensation in a temperature range of from 50 to 80°C and more especially 60°C-70°C at the same pressure as that of the evaporator to obtain, on the one hand, a liquid stream of Michael adducts, having a content of inhibitors of less than 1000 ppm, going to feed the cracker, and, on the other hand, a vapour stream, and
b. a condensation of this vapour stream in a temperature range of from 20°C to 40°C and more especially 20°C-30°C before taking up this liquid stream via a pump and feeding the rectification column.

8. Process according to any one of Claims 1 to 6, wherein the cracking is carried out at a temperature of 160°C to 180°C at atmospheric pressure.

9. Process according to any one of Claims 1 to 8, wherein the butyl acrylate obtained has a purity greater than 99.5%.

10. Process according to any one of Claims 1 to 9, wherein the polymerization inhibitor content in the bottom stream of the rectification column containing the Michael adduct stream is less than 3%.
